Europäisches Patentamt

European Patent Office

Office européen des brevets

(11) Veröffentlichungsnummer: 0 321 653
A1

## EUROPÄISCHE PATENTANMELDUNG

(21) Anmeldenummer: 88113828.3

(22) Anmeldetag: 25.08.88

(51) Int. Cl.4: A61M 5/32

Die Bezeichnung der Erfindung wurde geändert (Richtlinien für die Prüfung im EPA, A-III, 7.3).

(30) Priorität: 25.09.87 DE 8712926 U

(43) Veröffentlichungstag der Anmeldung:
28.06.89 Patentblatt 89/26

(84) Benannte Vertragsstaaten:
AT BE CH DE ES FR GB IT LI LU NL SE

(71) Anmelder: B. Braun Melsungen AG
Carl-Braun Strasse
D-3508 Melsungen(DE)

(72) Erfinder: Haindl, Hans, Dr.
Forstgarten 22
D-3508 Melsungen(DE)

(74) Vertreter: Selting, Günther, Dipl.-Ing. et al
Patentanwälte von Kreisler, Selting, Werner
Deichmannhaus am Hauptbahnhof
D-5000 Köln 1(DE)

(54) Nadelschutzkappe.

(57) Bei der erfindungsgemäßen Schutzkappe (130) ist vorgesehen, daß die radialen Vorsprünge (141) an der dem Kegeltrichter (137) zugewandten Außenseite eine zur Längsachse der Hülse (131) in Umfangsrichtung schräg verlaufende Gleitflanke (142) für die Längsstege (151) des Kanülenansatzes (150) aufweisen, die zwischen den radialen Vorsprüngen (141) klemmend an die Wandungsinnenfläche angreifen.

Je nach Orientierung der in Umfangsrichtung schrägverlaufenden Gleitflanke (142) jedes radialen Vorsprunges (141) bewirkt die Drehung der mit dem Kanülenansatz (150) lose zusammengesteckten Schutzkappe (130) in die eine oder andere Richtung eine Klemmverriegelung von Schutzkappe (130) und Kanülenansatz (150) durch gegenseitige Blockierung der radialen Vorsprünge (141) und Längsstege (151) einerseits bzw. ein axiales Wegschieben der Schutzkappe (130) von dem Kanülenansatz (150) andererseits. Durch Keilwirkung des die schräge Gleitflanke (142) übergleitenden Längssteges (151) des Kanülenansatzes (150) wird die Haftung zwischen den Längsstegen (151) und der Wandungsinnenfläche ohne die Verbindung von Spritzenkonus und Kanülenansatz (150) gefährdende Zugausübung aufgehoben, und die Kanüle (155) ist zur Benutzung frei.

FIG.3

## Schutzkappe

Die Erfindung bezieht sich auf eine Schutzkappe zur Verbindung mit einem Kanülenansatz einer Kanüle, bestehend aus einer Hülse mit rohrförmiger Wandung, die an einem Ende eine axiale Öffnung aufweist, von der ein nach außen erweiterter Kegeltrichter ausgeht und in deren Bereich auf der Wandungsinnenfläche radiale Vorsprünge ausgebildet sind, die bei Drehung der Hülse mit Längsstegen an dem Kanülenansatz zusammenwirken.

Einer der häufigsten Berufsunfälle ist das Stechen mit einer gebrauchten Kanüle mit der Folge von Infektionen. Die Unfallverhütungsvorschriften sehen daher vor, daß Kanülen-Schutzkappen ohne Kegeltrichter nach Gebrauch der Kanüle nicht wieder aufgeschoben werden dürfen, weil das Wiederaufschieben von solchen Schutzkappen eine besonders riskante Verrichtung ist. Die Unfallverhütungsvorschriften empfehlen deshalb, die gebrauchte Kanüle ohne Schutzkappe sofort in ein spezielles Kanülenabwurfgefäß abzuwerfen. Diese Empfehlung ist aus mehreren Gründen fragwürdig:
Es stehen nicht überall Kanülenabwurfgefäße dort, wo ein Arzt Injektionen macht oder Blut abnimmt, und die Unfallgefahr beim Transport der ungeschützten Kanüle zu einem Kanülenabwurfgefäß ist groß;
zum Abnehmen der ungeschützten Kanüle von der Spritze und zum Abwerfen der Kanüle in ein Kanülenabwurfgefäß ist es zumeist nötig, den Kanülenansatz anzufassen, wobei die Gefahr besteht, daß die Finger mit Patientenmaterial in Berührung kommen;
das Abwerfen der nicht geschützten Kanülen in beliebige Abfallbehälter, z.B. Beutel, gefährdet das Entsorgungspersonal hochgradig.

Die Schutzkappe der eingangs erwähnten Art, die in US-A-4 610 667 beschrieben ist, verringert das Sicherheitsrisiko beim Zusammenstecken mit der gebrauchten Kanüle dadurch, daß der Kegeltrichter die scharfe Kanülenspitze in die Öffnung der Hülse einführt, so daß mit verringerter Zielgenauigkeit des Anwenders Stichverletzungen der die Schutzkappe haltenden Hand mit einer benutzten Kanüle verhindert werden. Außerdem schützt der Kegeltrichter bei Annäherung der gebrauchten Kanüle gegen von dieser tropfendes infiziertes Fluid. Zum Zusammenhalten von Kanülenansatz und Schutzkappe bei der Entsorgung dienen radial einwärts gerichtete elastische Greifarme, die nach Widerhakenart an den Kanülenansatz angreifen und ein axiales Auseinanderziehen beider Teile verhindern. Diese Ausbildung verlangt eine spezielle Maßnahme zur Ermöglichung der Trennung der Schutzkappe von der unbenutzten Kanüle. Es sind zu diesem Zweck abbrechbare Laschen zwischen Kegeltrichter und Kanülenansatz geformt, die den Kanülenansatz außerhalb der Reichweite der Greifarme halten und die zerbrochen werden müssen, um die Schutzkappe abziehen zu können. Dies geschieht bei mit einem Spritzenkonus zusammengestecktem Kanülenansatz durch kräftige Drehung der Schutzkappe. Dies ist umständlich. Außerdem ist nachteilig, daß durch versehentliche axiale Krafteinwirkung die Schutzkappe gegen den Kanülenansatz geschoben werden kann, so daß die Greifarme wirksam werden und die Schutzkappe nicht abziehbar ist. Die Kanüle-Schutzkappenanordnung wird unbrauchbar und muß ersetzt werden. Auch können beim Abbrechen der Laschen Kunststoffpartikel freikommen, die ungünstigenfalls an der Kanüle haften und den Patienten gefährden. Die Abnahme der gebrauchten Kanüle mit der wiederaufgesetzten Schutzkappe von dem Spritzenkonus wird dadurch erleichtert, daß die Längsstege des Kanülenansatzes zwischen die aus achsparallelen Längsflanschen gebildeten Vorsprünge auf der Wandungsinnenfläche der Schutzkappe ragen und bei Drehung der Schutzkappe auf dem Kanülenansatz gegen die Längsflansche anschlagen, so daß die Schutzkappe drehstabil auf dem Kanülenansatz arretiert wird und als Mittel zur berührungslosen, kontaminationsgeschützten Abnahme der Kanüle von dem Spritzenkonus dient.

Der Erfindung liegt die Aufgabe zugrunde, die Schutzkappe der eingangs erwähnten Art so zu verbessern, daß das axiale Abziehen der Schutzkappe von der unbenutzten, an einer Spritze befestigten, Kanüle erleichtert wird.

Diese Aufgabe wird erfindungsgemäß dadurch gelöst, daß die radialen Vorsprünge an der dem Kegeltrichter zugewandten Außenseite eine zur Längsachse der Hülse in Umfangsrichtung schräg verlaufende Gleitflanke für die Längsstege des Kanülenansatzes aufweisen, die zwischen radialen Vorsprüngen klemmend an die Wandungsinnenfläche angreifen.

Je nach Orientierung der in Umfangsrichtung schrägverlaufenden Gleitflanke jedes radialen Vorsprunges bewirkt die Drehung der mit dem Kanülenansatz lose zusammengesteckten Schutzkappe in die eine oder andere Richtung eine Klemmverriegelung von Schutzkappe und Kanülenansatz durch gegenseitige Blockierung der radialen Vorsprünge und Längsstege einerseits bzw. ein axiales Wegschieben der Schutzkappe von dem Kanülenansatz andererseits. Durch Keilwirkung des die schräge Gleitflanke übergleitenden Längssteges des Kanülenansatzes wird die Haftung zwischen den Längsstegen und der Wandungsinnenfläche ohne die Verbindung von Spritzenkonus und Kanülenansatz gefährdende Zugausübung aufgehoben, und die Kanüle ist zur Benutzung frei. Die radialen Vorsprünge erfüllen eine Doppelfunktion, die sowohl das gemeinsame Abziehen von Schutzkappe und Kanülenansatz vom Spritzenkonus nach der Benutzung als auch das Entfernen der Schutzkappe vom Kanülenansatz vor der Benutzung erleichtert. In beiden Fällen verhindert der Kegeltrichter, daß der Anwender mit dem Kanülenansatz in Berührung kommt, so daß er selbst und der Patient gegen Kontaminationen und Infektionen geschützt werden. Da die Gleitflanke jedes radialen Vorsprunges der Schutzkappe bei ihrer Drehung als automatischer Abstoßmechanismus für die Schutzkappe wirkt, ist die Trennkraft so klein, daß die Haltekraft zwischen den Verbindungsorganen des Kanülenansatzes und der Spritze garantiert ausreicht, um beide Teile bei der Abnahme der Schutzkappe zusammenzuhalten. Der Kanülenansatz kann beim Abziehen der Schutzkappe nicht versehentlich von der Spritze getrennt werden, und das Einschleppen von Keimen in den Medikamentenweg durch mehrfaches Aufsetzen der Kanüle unterbleibt. Die sterile Medikamenten-Applikationsvorrichtung bleibt geschlossen und keine Kontaminationen dringen in das Medikament ein.

Die Längsstege des Kanülenansatzes können parallel zu seiner Längsachse verlaufen und mit einer der Kanüle zugewandten quergerichteten Stufe versehen sein, die mit der Gleitflanke des radialen Vorsprunges der Schutzkappe zusammenwirkt. Alternativ kann jeder Längssteg des Kanülenansatzes mit Steigung schräg zur Längsachse verlaufen. In diesem Falle greifen Längsstege und Gleitflanken nach der Art von Außen- und Innengewindeteilen zusammen und bewirken in einer Drehrichtung der Schutzkappe ihre Verklemmung mit dem Kanülenansatz gegen axiales Auseinanderziehen bzw. in der anderen Drehrichtung die axiale Auseinanderschiebung der beiden Teile.

In vorteilhafter Ausgestaltung ist vorgesehen, daß die Gleitflanke unter einem Winkel von 15° bis 45°, vorzugsweise 20° bis 30°, zur Längsachse der Hülse angestellt ist. Bei schrägen Längsstegen kann ihr Steigungswinkel dem Winkel des Verlaufes der Gleitflanken entsprechen.

Erfindungsgemäß kann jeder radiale Vorsprung als schräge Rippe mit zwei parallelen Längsrändern ausgebildet sein, von denen der äußere die schrägverlaufende Gleitflanke bildet. Die Anzahl der Rippen entspricht der Anzahl der Längsstege. Es sind mindestens zwei, vorzugsweise vier Paarungen vorgesehen, die gleichmäßig über die Umfänge von Schutzkappe und Kanülenansatz verteilt sind.

In weiterer Ausgestaltung der Erfindung ist vorgesehen, daß jeder radiale Vorsprung als Axialprofilierung mit einer zur Längsachse der Hülse parallelen Anschlagkante ausgebildet ist und daß der äußere Querrand der Axialprofilierung die schrägverlaufende Gleitflanke bildet. In diesem Falle wird durch die achsparallele Anschlagkante der Axialprofilierung eine vergrößerte Klemmfläche für den achsparallelen Längssteg des Kanülenansatzes geschaffen, so daß bei Drehung die Haftreibung zwischen Schutzkappe und Kanülenansatz beträchtlich erhöht wird, und zuverlässig Schutzkappe und kontaminierte Kanüle gemeinsam von der Spritze abgezogen und weggeworfen werden können. Die Innenfläche der Axialprofilierung kann von der Anschlagkante in Umfangsrichtung keilförmig abfallen. Hierdurch wird bei Drehung der Schutzkappe relativ zum Kanülenansatz ein geführtes Auflaufen der Längsstege auf die Gleitflanken erzielt. Zur weiteren Erleichterung der Handhabung sind die Axialprofilierungen in einem zylindrischen Ansatz vergrößerten Durchmessers der Hülse angeordnet, dessen Außenumfang eine Rändelung aufweist.

Bei dem vorzugsweise als Kreiskegeltrichter mit glatter Innenfläche gestalteten Kegeltrichter verläuft die Trichteröffnung vorzugsweise unter einem Winkel von etwa 45° zur Längsachse der Hülse. Die glatte Innenfläche bildet eine behinderungsfreie Führungsbahn für die auftreffende Kanülenspitze und die Abschrägung der Kegeltrichterwand unter einem Winkel von etwa 45° zur Längsachse der Hülse hat zur Folge, daß der Kegeltrichter sich auf mindestens den doppelten Durchmesser der Hülse erweitert, d.h., die Öffnung ist am äußeren Rand des Kegeltrichters etwa doppelt so groß wie an der Mündung der Hülse. Die axiale Länge der Wand des Kegeltrichters sollte mindestens dem halben Durchmesser der größten Öffnungsweite entsprechen. Der Verlängerung der Kegeltrichterwand über dieses Maß hinaus sind möglicherweise dadurch Grenzen gesetzt, daß die Verpackung der Kanüle mit Schutzkappe voluminös wird. Bei der erwähnten bevorzugten Abmessung wäre dieses Problem dadurch zu beheben, daß Kanüle und Schutzkappe jeweils lagenweise um eine Trichterlänge versetzt im Karton gestapelt werden, wodurch der Volumenzuwachs gering bleibt.

Die Wand des Kegeltrichters verläuft vorteilhafterweise gerade. Auch eine konvex nach innen gerichtete Wölbung würde dem Zweck der Einwärtsführung einer Kanülenspitze in Richtung des Hülsenhohlraumes entgegenkommen und ein solcher Trompetenkelch würde die Schutzwirkung des Kegeltrichters gegen ein Vorbeistecken der Kanülenspitze an der Hülsenöffnung verbessern.

Eine weitere Verbesserung kann dadurch erreicht werden, daß am äußeren Rand des Kegeltrichters ein radial nach außen gerichteter Flansch ausgebildet ist. In jedem Falle wird die die Schutzkappe haltende Hand nicht nur gegen Stichwunden und von der gebrauchten Kanüle herabtropfendes infiziertes Fluid abgeschirmt, sondern der Kegeltrichter schützt bei der erfindungsgemäß vereinfachten Abnahme der Schutzkappe von der Kanüle diese gegen Handberührung.

In der Zeichnung sind Ausführungsbeispiele der Erfindung schematisch dargestellt. Es zeigen:

Fig. 1 eine Kanüle und eine teilweise längsgeschnittene Ansicht einer Schutzkappe mit einer Ausführungsform der radialen Vorsprünge mit schräger Gleitflanke.

Fig. 2 eine abgewandelte Kanüle und den veränderten Kegeltrichterbereich einer weiteren Schutzkappe im Längsschnitt,

Fig. 3 eine Kanüle und einen Längsschnitt durch eine Schutzkappe mit einer zweiten Ausführungsform der radialen Vorsprünge mit schräger Gleitflanke und

Fig. 4 einen Querschnitt durch die Schutzkappe nach Fig. 3 längs der Linie IV-IV.

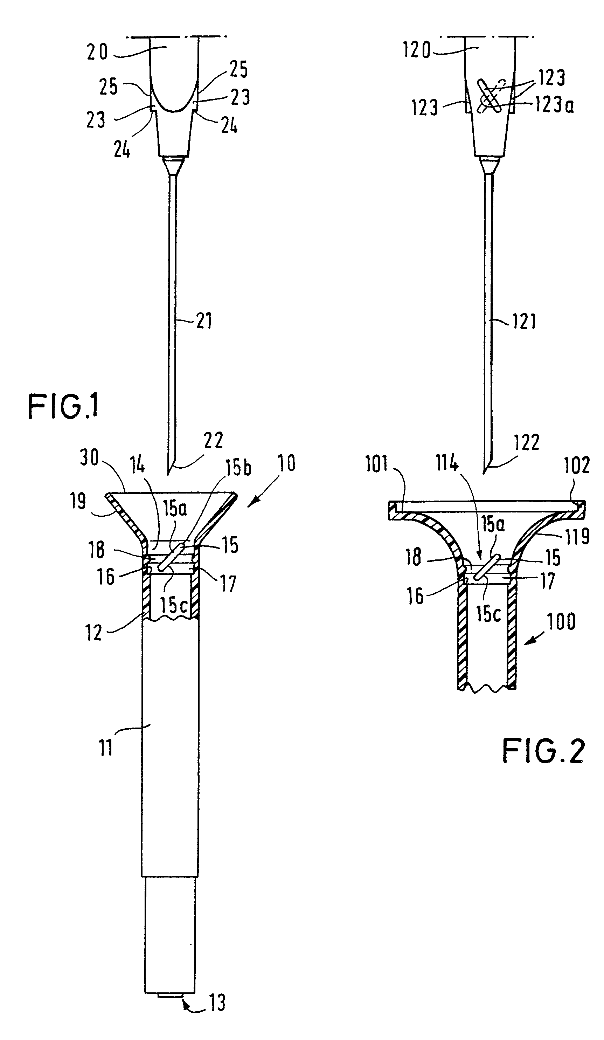

Gemäß Fig. 1 besteht eine Schutzkappe 10 für eine an einem hohlen Kanülenansatz 20 mit Innenkonus befestigte Injektionskanüle 21 mit scharf angeschliffener Spitze 22 aus einer im wesentlichen zylindrischen (oder profilierten) Hülse 11, deren rohrförmige Wandung 12 ringsum geschlossen ist und deren Bodenteil 13 geschlossen oder zur Ermöglichung der Sterilisierung des Hülseninnenraumes offen sein kann. Die gesamte Schutzkappe 10 ist als Kunststoff-Spritzteil ausgebildet und hat kreisförmigen Querschnitt. Das dem Bodenteil 13 entgegengesetzte Ende der geraden Hülse 11 ist mit einer axialen Öffnung 14 versehen, in deren unmittelbarer Nähe auf der Innenfläche der Wandung 12 mindestens zwei einander gegenüberliegende radiale Rippen 15 vorgesehen sind, die je eine in Umfangsrichtung gleichsinnig unter etwa 45° zur Längsachse der Schutzkappe 10 schräggestellte äußere Gleitfläche 15a aufweisen, zu der ein innerer Längsrand 15c parallel verläuft. Die Richtung jeder Rippe 15 ist - in Draufsicht auf die der Hülsenmitte zugewandte Fläche der Rippe 15 gesehen - von der Öffnung 14 (äußeres Ende 15b) nach links einwärts orientiert. Die geraden Rippen 15 haben gleichmäßige radiale Höhe und ihre Länge erstreckt sich von der Mündung der Öffnung 14 bis zu dem inneren Rand 16 einer Ringaussparung 17. An den oberen Rand der Ringaussparung 17 schließt sich eine radial nach innen gerichtete Ringwulst 18 an, die sich umfangsmäßig zwischen den Rippen 15 erstreckt. Die Ringwulst 18 hat im Querschnitt gesehen eine abgerundete Kuppe und sie dient als Gegen-Klemmorgan für den geraden achsparallelen Rand 25 von mindestens zwei auf dem Umfang des Kanülenansatzes 20 einander gegenüberliegende achsparallelen Längsstegen 23 zur klemmenden Zusammenhaltung der Schutzkappe 10 mit dem Kanülenansatz 20 vor und nach Gebrauch der Kanüle 21. Eine quergerichtete Stufe 24 jedes Längssteges 23 stößt gegen den inneren Rand 16 der Ringaussparung 17 an, so daß sich zur Verhinderung einer Beschädigung der Kanülenspitze 22 durch den Boden 13 der Schutzkappe 10 ein Anschlag für die Einschubtiefe ergibt.

Zum Abziehen der Schutzkappe 10 von der mit dem Konus einer Spritze verbunden bleibenden, zu benutzenden Kanüle 21 wird die Schutzkappe 10 leicht nach rechts (vom geschlossenen Ende gesehen) gedreht, wodurch die Stufe 24 jedes Längsstegs 23 die schräge äußere Gleitflanke 15a der zugeordneten Rippe 15 übergleitet und ein keilartiges Wegdrücken der Schutzkappe 10 von dem Kanülenansatz 20 erfolgt. Zur Trennung der benutzten Kanüle 21 von dem Spritzenkonus wird die Schutzkappe 10 wieder klemmend auf den Kanülenansatz 20 aufgesteckt und nach links (vom geschlossenen Ende gesehen) gedreht. Dabei legen sich die Seitenflächen der Längsstege 23 gegen das äußere Ende 15b der Rippen 15 und vermitteln eine drehstabile Verbindung von Kanülenansatz 20 und Schutzkappe 10, so daß die Schutzkappe 10 auf dem Kanülenansatz 20 arretiert ist. Bei der Linksdrehung wird das Drehmoment voll übertragen, und der Kanülenansatz 20 löst sich leicht vom Spritzenkonus, und die Kanüle 21 bleibt in der Schutzkappe 10. Dadurch wird es unnötig, daß der Kanülenansatz 20, der möglicherweise kontaminiert ist, zur Trennung von Spritze und Kanüle 21 vom Anwender mit den Fingern berührt wird. Wenn Spritze und Kanülenansatz 20 nicht über Konusverbindungen zusammengesteckt, sondern über Rechtsgewindeteile zusammengeschraubt sind, sollte die Schrägstellung der Rippen 15 zu der Darstellung in Fig. 1 entgegengesetzt vorgesehen sein, damit bei Drehung der Schutzkappe 10 zu ihrer Abnahme vom Kanülenansatz 20 dieser mit dem Spritzenkonus verbunden bleibt.

Die Wandung 12 der Hülse 11 der Schutzkappe 10 erweitert sich an der Öffnung 14 nach außen und geht in einen kreisförmigen Kegeltrichter 19 über. Der Kegeltrichter 19 öffnet sich im Winkel von etwa 45° auf mindestens den doppelten Durchmesser der Hülse 11. Vorzugsweise hat die Öffnung 30 des Kegeltrichters 19 an ihrer größten Stelle einen Durchmesser von 17 mm und die axiale Länge der Wand des Kegeltrichters 19 beträgt ca. 6 mm. Die Wand des Kegeltrichters 19 ist innen glattflächig, damit bei Anstoßen der Spitze 22 der Kanüle 21 gegen die Trichterfläche die Spitze 22 hindernislos in die Öffnung 14 hineingeleitet wird. Die gebrauchte Kanüle 21 kann in der Schutzkappe 10 steckend mit dieser von einer Spritze abgenommen werden, so daß das Berühren des Kanülenansatzes 20 und die damit verbundene Infektionsgefahr für den Anwender entfällt. Auch hierbei übt der ausgestellte Kegeltrichter 19 eine Schutzwirkung für den Anwender aus.

Bei dem Beispiel nach Fig. 2 ist eine Schutzkappe 100 an ihrer Öffnung 114 mit einer anderen Ausführungsform eines Kegeltrichters 119 versehen. In diesem Falle ist die Wand des Kegeltrichters 119 konvex nach innen gewölbt, so daß sich eine gebogene Kelchform ergibt, auf deren Innenfläche die Spitze 122 einer Kanüle 121 in die Öffnung 114 der Schutzkappe 100 gleitet. Zusätzlich wird die Schutzwirkung des in Draufsicht kreisförmigen Kegeltrichters 119 dadurch erhöht, daß der Rand 101 als nach außen gerichteter radialer Flansch verlängert ist und daß die äußere Kante des Randes 101 als nach oben ragende Umrandung 102 ausgebildet ist, die eine weitere Abrutschsicherung für die Kanülenspitze 122 bildet. Die Ausstattung der Innenseite der Öffnung 114 der Schutzkappe 100 entspricht derjenigen der Öffnung 14 des Beispieles der Fig. 1. Mit dieser Verriegelungs- und Freigabeanordnung der Schutzkappe 100 wirkt ein abgewandelter Kanülenansatz 120 der Kanüle 121 zusammen. Anstatt der achsparallelen Längsstege 23 des Kanülenansatzes 20 sind zur Längsachse schräggestellte Längsstege 123 vorgesehen, die in gleicher Richtung wie die Rippen 15 orientiert sind. Auch die Winkelanstellung der Längsstege 123 entspricht derjenigen der Rippen 15. Bei Drehung des mit Hilfe der Ringwulst 18 mit dem Kanülenansatz 120 klemmend zusammensteckenden Schutzkappe 100 nach rechts (vom geschlossenen Ende gesehen) übergleitet die untere Schrägkante 123a jedes schrägen Längssteges 123 die nach oben weisende Gleitflanke 15a jeder Rippe 15, und die Schutzkappe 100 wird von dem Kanülenansatz 120 axial weggedrückt. Bei entgegengesetzter Drehung der Schutzkappe 100 werden die schrägen Flächen 15a und 123a jeweils zusammengepreßt, das Drehmoment wird voll übertragen, der mit der Schutzkappe 100 verbunden bleibende Kanülenansatz 120 löst sich leicht vom Spritzenkonus und kann risikolos entsorgt werden.

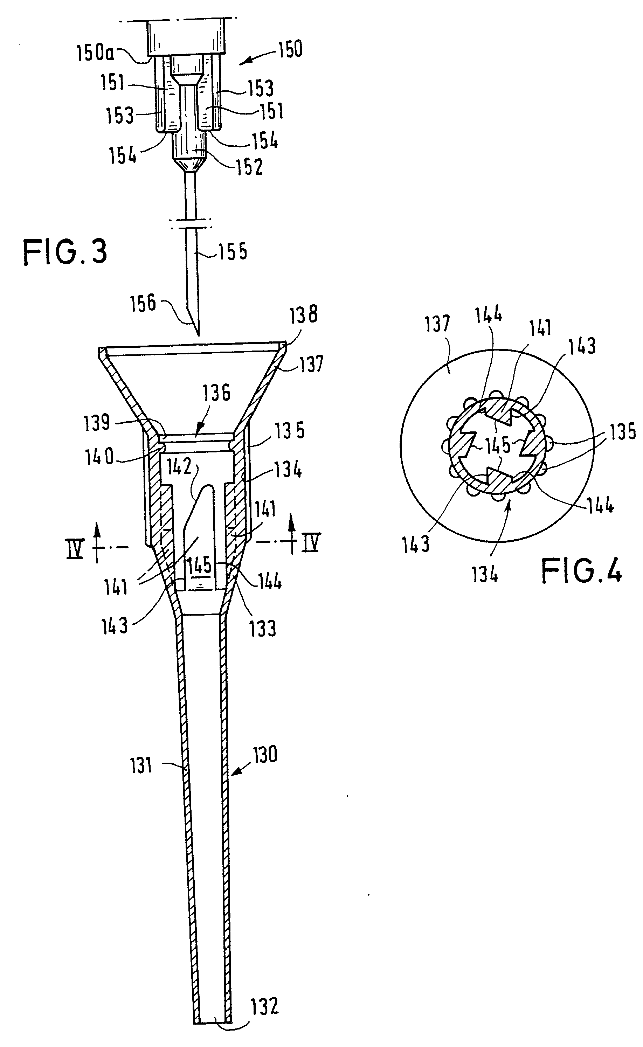

Das Prinzip der Ausführungsform nach Fign. 3 und 4 entspricht demjenigen der Fign. 1 und 2. Eine Schutzkappe 130 aus steifem Kunststoffmaterial besteht aus einer rohrförmigen Hülse 131, die an ihrem unteren Ende 132 zur Sterilisierung des Hülseninnenraumes offen ist und die am anderen Ende über eine kegelförmige Erweiterung 133 in einen zylindrischen Ansatz 134 übergeht, dessen Innendurchmesser größer ist als der Innendurchmesser der Hülse 131. Der Ansatz 134 ist auf der Außenfläche mit einer Rändelung 135 versehen, die die Handhabung der Schutzkappe 130 erleichtert. An eine kreisförmige Öffnung 136 des Ansatzes 134 schließt sich ein nach außen erweiterter Kegeltrichter 137 mit kreisförmigem Querschnitt an. Der obere Rand 138 des Kegeltrichters 137 ist zylindrisch hochgezogen. Am Übergang zwischen Kegeltrichter 137 und Ansatz 134 ist eine zylindrische Stufe 139 ausgebildet, die auf der Innenseite von einer radialen Ringwulst 140 begrenzt ist. Unterhalb der Ringwulst 140 sind mit gewissem axialen Abstand, jedoch noch im Bereich der Öffnung 136, vier Axialprofilierungen 141 in Form gerader Leisten ausgebildet, die auf der Innenfläche des Ansatzes 134 radial nach innen gerichtet sind und an ihrem dem Kegeltrichter 137 zugewandten Ende eine schräg verlaufende Gleitflanke 142 aufweisen, während ihr anderes Ende an dem Übergang der kegelförmigen Erweiterung 133 flach auslaufend endet. Jede Axialprofilierung 141 ist infolge der schrägen Gleitflanke 142 mit einer Anschlagkante 143 und einem zu dieser parallelen längeren Längsrand 144 versehen.

Jede Axialprofilierung 141 ist auf der einwärts gerichteten Innenfläche 145 in Umfangsrichtung keilförmig abgeschrägt, wie Fig. 4 zeigt. Die Abschrägung fällt von der kürzeren Anschlagkante 143 gegen den Längsrand 144 gleichmäßig ab. Die Gleitflanke 142 jeder Axialprofilierung 141 ist unter einem Winkel von 30° zur Längsachse der Schutzkappe 130 schräggestellt, und zwar in Draufsicht auf die der Hülsenmitte zugewandte Fläche 145 gesehen - von der Öffnung 136 nach links einwärts orientiert.

Ein Kanülenansatz 150, der zu der Schutzkappe 130 paßt, ist mit einem üblichen, nicht dargestellten, Innenkonus zur Verbindung mit dem Außenkonus einer Spritze versehen und weist vier mit gleichmäßigen Abständen über seinen Umfang verteilte Längsstege 151 auf. Jeder Längssteg 151 ist von einer zentralen Nabe 152 radial nach außen gerichtet und mit einem geraden achsparallelen Rand 153 sowie einer zur Längsachse des Kanülenansatzes 150 quergerichteten Stufe 154 versehen. In die Nabe 152 ist koaxial eine Stahlkanüle 155 mit angeschliffener Spitze 156 eingelassen.

Wenn der Kanülenansatz 150 mit der Schutzkappe 130 zusammengesteckt ist, stößt ihr Rand 150a gegen die Außenfläche der Ringwulst 140 der Schutzkappe 130 an, wodurch gewährleistet ist, daß die Spitze 156 der Kanüle 155 nicht über das offene Ende 132 der Schutzkappe 130 nach außen vorsteht. Die Ränder 153 liegen gegen die Innenfläche des Ansatzes 134 zwischen den Axialprofilierungen 142 fest an, so daß durch Haftreibung ein Zusammenhalt zwischen Kanülenansatz 150 und Schutzkappe 130 gewährleistet ist, und zwar sowohl vor als auch nach der Benutzung der Kanüle 155. Um die Schutzkappe 130 von dem mit einem Spritzenkonus zusammengesteckten Kanülenansatz 150 problemlos abnehmen zu können, wird die Schutzkappe 130 (vom geschlossenen Ende gesehen) nach rechts gedreht, so daß die Stufen 154 der Längsstege 151 über die erhabenen Gleitflanken 142 der Axialprofilierungen 141 gleiten und ein keilartiges axiales Wegschieben der Schutzkappe 130 von dem Kanülenansatz 150 erfolgt. Zur berührungslosen Trennung der benutzten Kanüle 155 von dem Spritzenkonus wird die Schutzkappe 130 auf den Kanülenansatz 150 wieder aufgesteckt, und es wird durch - vom geschlossenen Ende gesehen - linksgerichtete Drehung der Schutzkappe 130 von dem flachen Längsrand 144 her eine feste Anpressung des Randes 153 jedes Längssteges 151 auf die kreisförmige Innenfläche 145 jeder Axialprofilierung 141 erzielt, die durch Erhöhung der Haftreibung zwischen den Teilen und Drehstabilisierung relativ zueinander Kanülenansatz 150 und Schutzkappe 130 so miteinander verriegelt, daß beide Teile gemeinsam leicht von dem Spritzenkonus abgezogen werden können.

## Ansprüche

1. Schutzkappe (10) zur Verbindung mit einem Kanülenansatz (20) einer Kanüle (21), bestehend aus einer Hülse (11) mit rohrförmiger Wandung (12), die an einem Ende eine axiale Öffnung (14) aufweist, von der ein nach außen erweiterter Kegeltrichter (19) ausgeht und in deren Bereich auf der Wandungsinnenfläche radiale Vorsprünge ausgebildet sind, die bei Drehung der Hülse (11) mit Längsstegen (23) an dem Kanülenansatz (20) zusammenwirken,
**dadurch gekennzeichnet,**
daß die radialen Vorsprünge (15) an der dem Kegeltrichter (19) zugewandten Außenseite eine zur Längsachse der Hülse (11) in Umfangsrichtung schrägverlaufende Gleitflanke (15a) für die Längsstege (23) des Kanülenansatzes (20) aufweisen, die zwischen radialen Vorsprüngen (15) klemmend an die Wandungsinnenfläche angreifen.

2. Schutzkappe nach Anspruch I, dadurch gekennzeichnet, daß die Gleitflanke (15a) unter einem Winkel von 15° bis 45°, vorzugsweise 20° bis 30°, zur Längsachse der Hülse (11) angestellt ist.

3. Schutzkappe nach Anspruch 1 oder 2, dadurch gekennzeichnet, daß jeder radiale Vorsprung als schräge Rippe (15) mit zwei parallelen Längsrändern ausgebildet ist, von denen der äußere die schrägverlaufende Gleitflanke (15a) bildet.

4. Schutzkappe nach Anspruch I oder 2, dadurch gekennzeichnet, daß jeder radiale Vorsprung als Axialprofilierung (141) mit einer zur Längsachse der Hülse (131) parallelen Anschlagkante (143) ausgebildet ist, und daß der äußere Querrand der Axialprofilierung (141) die schrägverlaufende Gleitflanke (142) bildet.

5. Schutzkappe nach Anspruch 4, dadurch gekennzeichnet, daß die Innenfläche (145) der Axialprofilierung (141) von der Anschlagkante (143) in Umfangsrichtung keilförmig abfällt.

6. Schutzkappe nach Anspruch 4 oder 5, dadurch gekennzeichnet, daß die Axialprofilierungen (141) in einem zylindrischen Ansatz (134) vergrößerten Durchmessers der Hülse (131) angeordnet sind, dessen Außenumfang eine Rändelung (135) aufweist.

7. Schutzkappe nach einem der Ansprüche 1 bis 6, dadurch gekennzeichnet, daß jeder Längssteg (23) des Kanülenansatzes (20) parallel zur Längsachse verläuft und mit einer der Kanüle (21) zugewandten, quergerichteten Stufe (24) versehen ist.

8. Schutzkappe nach einem der Ansprüche 1 bis 6, dadurch gekennzeichnet, daß jeder Längssteg (151) des Kanülenansatzes (150) mit Steigung schräg zur Längsachse verläuft.

FIG.1

FIG.2

EP 0 321 653 A1

FIG.3

FIG.4

**EUROPÄISCHER RECHERCHENBERICHT**

## EINSCHLÄGIGE DOKUMENTE

EP 88113828.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| D,Y | US - A - 4 610 667 (J. PEDICANO et al.)<br><br>* Fig. 2,3,6-11; Zusammenfassung *<br><br>-- | 1,4,6, 7 | A 61 M 5/32 |
| Y | US - A - 4 237 882 (R. WICKHAM)<br><br>* Gesamt; insbesondere Fig. 4-6; Zusammenfassung *<br><br>---- | 1,4,6, 7 | |

RECHERCHIERTE
SACHGEBIETE (Int. Cl.4)

A 61 M 5/00

Der vorliegende Recherchenbericht wurde für alle Patentansprüche erstellt.

| Recherchenort | Abschlußdatum der Recherche | Prüfer |
|---|---|---|
| WIEN | 17-02-1989 | LUDWIG |